# NOUVEAU FASCICULE DE BREVET EUROPEEN

(11) **EP 0 569 309 B2**
(45) Date de publication et mention de la décision concernant l'opposition: **16.01.2008**
(45) Mention de la délivrance du brevet: 11.08.1999
(21) Numéro de dépôt: 93420183.1
(22) Date de dépôt: 06.05.1993
(51) Int. Cl.: C07K 14/18, A61K 39/29, G01N 33/576, C07K 16/10

(54) **Polypeptides de synthèse appartenant au virus de l'hépatite C (VHC) et utilisables notamment pour détecter ce dernier**
Synthetische Polypeptide vom Hepatitis C-Virus, verwendbar für den Nachweis des Virus
Hepatitis C virus synthetic polypeptides usable for detection of the virus

(30) Priorité: 06.05.1992 FR 9205763
(43) Date de publication de la demande: 10.11.1993
(73) Titulaire: BIO MERIEUX, 69280 Marcy l'Etoile (FR)
(72) Inventeur: Dalbon, Pascal, F-69200 Venissieux (FR); Jolivet, Michel, F-69500 Bron (FR)
(74) Mandataire: Guerre, Dominique

(56) Documents cités:
- EP-A- 0 442 394
- EP-A- 0 484 787
- EP-A- 0 525 910
- WO-A-92/22571
- PROC. NATL ACAD. SCI. USA vol. 88, no. 12, 1991, pages 5462 - 5466 M. NASOFF ET AL. 'Identification of immunodominant epitope within the capsid protein of hepatitis C virus'
- PROC. NATL ACAD. SCI. USA vol. 88, no. 9, 1991, pages 3647 - 3651 B. HOSEIN ET AL. 'Improved serodiagnosis of hepatitis C virus infection with synthetic peptide antigen from capsid protein'
- Y. SHIMONISHI (ED) 'Peptide Chemistry 1990' 1991 , PROTEIN RESEARCH FOUNDATION , OSAKA E. Munekata et al., Epitope mapping of hepatitis C constituting protein

## Description

La présente invention concerne de manière générale des polypeptides de synthèse, c'est-à-dire obtenus par des voies préparatives telles que synthèse chimique, composés d'acides aminés consécutifs, identiques ensemble à tout fragment, séquence, ou région de la protéine structurale de la nucléocapside dite protéine CORE du virus humain de l'hépatite C (VHC). Ces polypeptides peuvent être utilisés comme antigènes de synthèse, dans différentes applications découlant de leur pouvoir immunogène, et précisées ci-après; au premier rang de ces applications figure la détection du VHC, dans différents milieux corporels, par exemple un échantillon ou prélèvement sanguin.

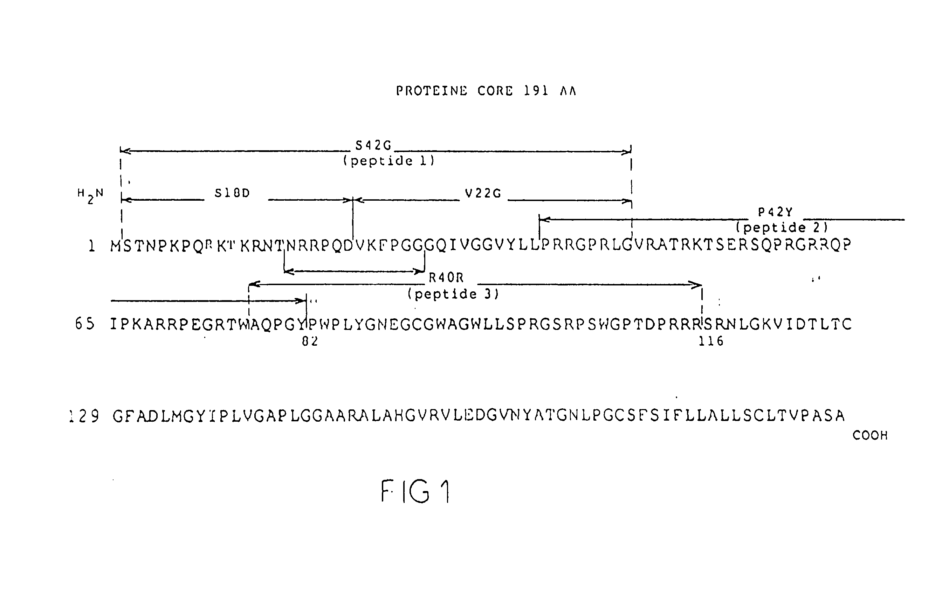

Il a été établi que la protéine de la nucléocapside, ou protéine CORE, du VHC, se composant comme établi par la figure 1 de 191 acides aminés, est celle qui présente l'homologie la plus importante, d'une part entre les séquences d'un même groupe d'isolats viraux, et d'autre part entre les différents groupes de virus. Par ailleurs, cette protéine CORE est codée par une partie structurale du génome du VHC, et constitue donc une protéine structurale. La grande conservation de la structure de cette protéine en fait un candidat, particulièrement adapté à la détection immunologique du VHC.

Ainsi les travaux de Hosein B, Fang CT, Popovsky MA, Ye J, Zhang M, Wang CY, publiés dans Improved serodiagnosis of hepatitis C virus infection with synthetic peptide antigen from capsid protein. Proc Natl Acad Sci USA 1991; 88: 3647-51, ont permis de déterminer une région immunodominante, dans la protéine CORE, correspondant à un polypeptide constitué par la séquence des acides aminés 1 à 120 N-terminaux de ladite protéine CORE.

Conformément à une publication déjà citée, à savoir : Hosein B, Fang CT, et al, Improved serodiagnosis of hepatitis C virus infection with synthetic peptide antigen from capside protein. Proc Natl Acad Sci USA 1991; 88 : 3647-51, différents peptides synthétiques correspondant à certaines séquences de la protéine CORE peuvent être utilisés en tant qu'antigène dans des tests de détection en phase solide, par exemple sur des supports immunoabsorbants.

Dans le même but de détection immunologique du VHC, le document EP-0 442 394 décrit plusieurs polypeptides comprenant une séquence peptidique appartenant à la région immunodominante ci-dessus mentionnée, de la protéine CORE.

Parmi lesdits polypeptides, celui dit VIIIE constitué par la séquence des acides aminés 2 à 62 N-terminaux de la protéine CORE a été testé du point de vue de son immunoréactivité vis-à-vis des anticorps anti-VHC contenus dans des sérums d'individus infectés par le VHC, dans un test ELISA. Ce polypeptide a démontré une bonne immunoréactivité, visà-vis des sérums infectés par le VHC testés.

La substitution de tels polypeptides connus de l'art antérieur, obtenus par synthèse chimique, à la protéine de fusion correspondant à la protéine CORE proprement dite, dans des tests de détection, est avantageuse car elle permet de diminuer les risques d'immunoréaction avec des anticorps susceptibles d'être présents dans un échantillon et différents de ceux dirigés contre le VHC.

Cependant, il est apparu essentiel à la Demanderesse de pouvoir déterminer une séquence minimale et suffisante pour un polypeptide qui, du point de vue de ses propriétés antigéniques, soit équivalente à la protéine dans son entier.

En effet, plus un peptide est long, plus sont accrus les risques susceptibles de pertuber l'antigénicité dudit peptide en raison de la plus grande fréquence des événements suivants :
- interférence entre le peptide et des anticorps différents de ceux dirigés contre le VHC par réactions croisées ou entre le peptide et d'autres molécules biologiques présentes dans le milieu,
- modifications conformationnelles par rapport à la structure de la protéine native qui peuvent se traduire par une disparition de conformations secondaires et/ou tertiaires correspondant à des sites épitopiques, ou apparition de conformations secondaires et/ou tertiaires différentes de celles qu'adopte la protéine entière, susceptibles d'interagir avec des anticorps autres que les anticorps anti-VHC.

Selon EP-0 442 394, les inventeurs ont tenté de raccourcir la longueur du polypeptide VIIIE en l'amputant respectivement de 9, 19, 29 et 39 acides aminés dans sa partie N-terminale pour préparer les polypeptides constitués par les séquences d'acides aminés N-terminaux de la protéine CORE, respectivement de longueur 10 à 62, 20 à 62, 30 à 62 et 40 à 62.

L'immunoréactivité de chacun de ces peptides a été évaluée dans des tests ELISA et on observe que plus le nombre d'acides aminés amputés est grand, plus l'immunoréactivité diminue.

A l'encontre de ces résultats, la présente invention apporte un polypeptide, ou ses fragments, qui bien que constitué par une séquence d'acides aminés beaucoup plus courte que celle de la structure polypeptidique VIIIE, manifeste une immunoréactivité avec la totalité des sérums d'individus ou échantillons infectés par le VHC et porteurs d'anticorps dirigés contre la protéine de nucléocapside.

La présente invention a pour origine les découvertes totalement inattendues suivantes, résultant du protocole expérimental exposé ci-après :
**1)** dans la protéine CORE du VHC, il existe une région immunodominante représentée par au plus les 45 premiers acides aminés;
**2)** cette région immunodominante suffit à elle seule pour obtenir la même sensibilité qu'avec la protéine CORE totale, en ce qui concerne la détection des anticorps anti-VHC;
**3)** cette région immunodominante doit être continue, si on veut réagir avec la totalité des sérums d'individus ou échantillons sanguins infectés par le VHC et présentent des anticorps dirigés contre la protéine CORE;
**4)** cette région immunodominante comporte manifestement des épitopes de type conformationnel et des épitopes de type linéaire.

En conséquence, le polypeptide utilisé conformément à l'invention comprend une séquence peptidique isolée se composant environ des 45 acides aminés N-terminaux de la protéine CORE du virus VHC (cf SEQ ID N01).

Préférentiellement, le polypeptide de l'invention est constitué uniquement ou par une séquence peptidique isolée se composant des 45 acides aminés N-terminaux de ladite protéine ou alternativement par tout polypeptide homologue, comprenant environ 45 acides aminés, et présentant une réactivité antigénique vis-à-vis du VHC.

De manière encore préférentielle, le polypeptide de l'invention est constitué par une séquence peptidique se composant des acides aminés 2 à 45 N-terminaux de la protéine CORE (cf SEQ ID N02).

La présente invention découle des observations expérimentales, comme défini ci-après.

L'invention apporte notamment tout d'abord l'un quelconque des composés ou compositions polypeptidiques suivants :
a) uniquement une séquence peptidique isolée, se composant de 45 acides aminés N-terminaux de la protéine CORE (ou capside) du virus humain de l'hépatite C (VHC), telle que représentée à la Figure 1, cette séquence étant éventuellement amputée dans sa partie N-terminale et/ou sa partie C-terminale de 1 à 10 acides aminés;
b) uniquement une séquence peptidique, présentant une réactivité immunologique vis-à-vis des anticorps anti-VHC, SEQ ID NO.2;
c) uniquement une séquence homologue à la séquence peptidique (a) ou (b), dont chaque acide aminé constitutif présente des propriétés chimiques identiques ou analogues à celles de l'acide aminé homologue dans la séquence peptidique (a) ou (b);
d) tout mélange approprié des composés polypeptidiques selon (a) à (c).

Par "séquence peptidique isolée", on entend tout polypeptide non fusionné avec une autre protéine ou un autre peptide, quelle que soit sa voie d'obtention, par exemple par synthèse chimique, lyse de la protéine CORE, ou par des techniques de recombinaison génétique. Ce polypeptide peut par conséquent être un peptide de synthèse, ou une protéine.

Selon la présente invention, un acide aminé est dit homologue d'un autre acide aminé, lorsque leurs caractéristiques chimiques, telles que polarité, hydrophobicité, et/ou basicité, et/ou acidité, et/ou neutralité, sont sensiblement les mêmes. Ainsi, une leucine est homologue d'une isoleucine, au sens de la définition précédente.

Les séquences polypeptidiques selon l'invention peuvent être telles qu'à l'état natif, ou modifiées chimiquement. Par modification chimique, on entend toute altération chimique d'au moins un groupement fonctionnel de la séquence peptidique, préservant substantiellement voire développant les propriétés biologiques de ladite séquence. Font notamment partie des modifications chimiques considérées précédemment, le remplacement d'un acide aminé de la série L par un acide aminé de la série D, une modification des chaînes latérales des acides aminés, telle qu'une acétylation des fonctions amine, une carboxyméthylation des fonctions thiols, une estérification des fonctions carboxyliques, une modification des liaisons peptidiques telles que des liaisons carba, retro-inverso, réduites et méthylène-oxy.

A partir des composés ou compositions peptidiques définis précédemment, l'invention propose un réactif de détection du virus humain de l'hépatite C (VHC), comprenant à titre de substance réactive l'un quelconque des composés ou compositions précités, et éventuellement tout additif immuno-compatible avec la détection du VHC. Ainsi, la détection peut être effectuée à l'aide d'un polypeptide identique ou analogue à ceux de la présente invention avec éventuellement un anticorps anti-immunoglobuline humaine, marqués par tout marqueur conventionnel, tel que marqueur radioactif, fluorescent, enzymatique ou analogue. Un tel réactif peut être utilisé, aussi bien en phase homogène, par exemple dans des essais d'immuno-précipitation, qu'en phase hétérogène, par exemple dans des essais d'immuno-adsorption.

Avec le réactif précité, on peut obtenir tout moyen approprié de détection du VHC, qu'il s'agisse d'un kit de détection ou de tout autre système ou ensemble équivalent. A titre d'exemple, le réactif précité est supporté sur un support solide, immuno-compatible avec le réactif dans son ensemble; en particulier le support solide est sans limitation sous la forme d'une plaque de micro-titration, d'une feuille, d'un cône, d'un puits, d'une bille, ou tout autre substrat micro-particulaire approprié.

Le terme support solide tel qu'utilisé ici inclut tous les matériaux sur lesquels peuvent être immobilisés les polypeptides selon l'invention. Ce peut être des matériaux de synthèse modifiés ou non chimiquement, notamment des polysaccharides, tels que les matériaux de cellulose, par exemple du papier, des dérivés de cellulose tels que la nitrocellulose et l'acétate de cellulose; des polymères tels que chlorure de vinyle, polyéthylène, polystyrène, polyacrylate, ou copolymères tels que polymère de chlorure de vinyle et de propylène, polymère de chlorure de vinyle et d'acétate de vinyle; copolymères à base de styrène; des fibres naturelles telles que le coton et des fibres synthétiques telles que le nylon. De préférence, le support solide est un polymère de polystyrène, un copolymère butadiène-styrène ou un copolymère butadiène-styrène en mélange avec un ou plusieurs polymères ou copolymères choisis parmi le polystyrène, les copolymères styrène-acrylonitrile ou styrène-méthylméthacrylate de méthyle, les polypropylènes, les polycarbonates ou analogues.

A partir des réactifs ou moyens de détection immunologique selon l'invention, on peut détecter des anticorps anti-VHC, dans toute partie ou milieu corporel, tel qu'un échantillon du sang d'un individu suspecté d'être infecté par le VHC. Il suffit pour cela de mettre en contact cette partie corporelle et le réactif précité, dans des conditions prédéterminées, par exemple de température, permettant une éventuelle réaction immunologique, et de détecter ensuite la présence d'un complexe immun avec ce réactif.

Par "partie corporelle", on entend tout fluide, tissu ou organe d'un individu, comprenant ou susceptible de comprendre des anticorps anti-VHC. Ces parties corporelles peuvent être un échantillon de sang, de plasma, de sérum, des sécrétions diverses, etc...

Le procédé décrit précédemment peut être mis en oeuvre dans tout dispositif ou appareil de détection, comprenant un récipient de mise en contact de la partie corporelle analysée, avec un réactif tel que défini précédemment, et ce avec des moyens créant des conditions, telles que température, propices à une éventuelle réaction immunologique. Et ce dispositif comprend des moyens de détection, notamment optiques, du complexe immun obtenu avec le réactif.

Une autre manière de détecter le virus VHC à partir des polypeptides selon la présente invention est d'obtenir des anticorps monoclonaux ou polyclonaux, par toute technique connue en elle-même, comprenant une réaction immunologique entre un organisme humain ou animal et un agent immunogène constitué par une composition polypeptidique telle que définie précédemment. Les anticorps ainsi obtenus, par exemple convenablement marqués, peuvent être utilisés pour détecter le VHC, ou pour suivre la progression du virus chez un malade atteint de l'hépatite C.

Le caractère immuno-dominant de la séquence peptidique selon la présente invention, a été mis en évidence conformément au protocole expérimental suivant.

La stratégie choisie consiste à synthétiser des longs fragments peptidiques, d'environ 40 acides aminés, dans la partie N-terminale de la protéine CORE, appartenant à la séquence des environ 120 premiers acides aminés.

On a donc dans un premier temps défini trois peptides, en commençant la synthèse à l'acide aminé n°2 (Serine).

Conformément à la figure 1, trois peptides ont été synthétisés, à savoir :
- peptide dit S42G, s'étendant de la sérine 2 jusqu'à la glycine 45
- peptide dit P42Y, s'étendant de la proline 39 jusqu'à la tyrosine 82
- peptide dit R40R, s'étendant de l'arginine 75 jusqu'à l'arginine 116.

Il apparaît que ces peptides présentent quelques acides aminés en commun, ce qui permet de mettre en évidence un éventuel déterminant antigénique localisé à l'intersection de deux peptides.

Les peptides ont été synthétisés chimiquement par synthèse en phase solide, selon la technique de Merrifield (Barany G, and Merrifield R.B, 1980, In the Peptides, 2, 1-284, Gross E and Meienhofer J, Eds Academic Press, New York). Les modalités pratiques sont celles décrites ci-après.

### Synthèse des peptides

Les peptides sont synthétisés sur une résine phénylacétamidométhyle (PAM)/polystyrène/divinylbenzène (Applied Biosystems, Inc. Foster City, CA), en utilisant un synthétiseur automatique "Applied Biosystems 430A". Les acides aminés sont couplés sous forme d'esters d'hydroxybenzotriazole (HOBT). Les acides aminés utilisés proviennent de chez Novabiochem (Laüflerlfingen, Suisse) ou de chez BACHEM (Bubendorf, Suisse).

La synthèse chimique des peptides a été réalisée en utilisant un protocole de double couplage avec la N-méthyl-pyrrolidone (NMP) comme solvant. Les peptides ont été coupés de leur résine ainsi que les protections latérales, de manière simultanée, à l'aide d'acide fluor; hydrique (HF) dans un appareillage approprié, (appareil de coupure de type I, Peptide Institute, Osaka, Japon).

Pour lg de peptidylrésine, 10 ml de HF, 1 ml d'anisole et 1 ml de diméthylsulfure (DMS) sont utilisés, et le mélange est agité durant 45 minutes à -2°C. Le HF est alors évaporé sous vide. Après des lavages intensifs à l'éther, le peptide est élué de la résine par de l'acide acétique 10 %, puis le peptide est lyophilisé.

Les peptides sont purifiés par chromotographie liquide haute performance préparative sur une colonne VYDAC de type C18 (250 x 21 mm) (The Separation Group, Hesperia, CA, USA). L'élution est réalisée par un gradient d'acéto-nitrile à un débit de 22 ml/mn. Les fractions collectées sont contrôlées par une élution en condition isocratique sur une colonne VYDAC C18 analytique (250 x 4,6 mm), à un débit de lml/mn. Les fractions qui présentent le même temps de rétention sont réunies et lyophilisées. La fraction majoritaire est ensuite analysée par chromatographie liquide haute performance analytique, avec le système décrit précédemment. Le peptide qui est considéré comme de pureté acceptable se traduit par un pic unique représentant 95 % minimum du chromatogramme.

Les peptides purifiés sont analysés dans le but de contrôler leur composition en acides aminés, à l'aide d'un analyseur d'acides aminés automatique Applied Biosystems 420 H. La mesure de la masse moléculaire chimique (moyenne) des peptides est obtenue en utilisant la spectrométrie de masse L.S.I.M.S. en mode d'ion positif sur un instrument à double focalisation VG. ZAB.ZSEQ, relié à un système d'acquisition DEC-VAX 2000 (VG analytical Ltd, Manchester, Angleterre).

La réactivité de ces trois peptides vis-à-vis de sérums d'individus infectés par le virus de l'hépatite C dits (VHC) positifs, a été évaluée dans un test ELISA selon le protocole décrit ci-après.

### Détection des anticorps anti-VHC par ELISA

Les puits d'une plaque de microtitration de marque "NUNC maxisorb" sont saturés par 100 µl d'une solution contenant le peptide ou un mélange de peptides (10 µg/ml) durant 2 heures à 37°C. La plaque est ensuite vidée puis lavée à l'aide d'un tampon de lavage contenant 0,05 % de Tween 20. Les puits sont saturés par 100 µl de tampon de lavage additionné de 10 % de sérum de chèvre (v/v), puis incubés pendant 30 minutes à 37°C, puis lavés à nouveau comme précédemment. Les sérums à analyser sont dilués à la dilution appropriée avec le tampon de saturation. L'incubation des sérums est de 1 heure à 37°C. Les puits sont à nouveau lavés. La solution de conjugué (IgG de chèvre anti IgG humain marquées par la peroxydase) à une dilution de 1/1000 dans le tampon de saturation est alors ajoutée et l'incubation dure 90 minutes à 37°C. Après lavages, la solution de substrat orthophénylène-diamine est ajoutée. Après 10 minutes, la réaction est stoppée par 50 µl d'H₂SO₄ et la densité optique est lue à 492 nm. Il est à noter que tous les tests ont été réalisés en double.

La réactivité des peptides S42G, P42Y, et R40R est mesurée en ELISA, vis-à-vis de sérums VHC positifs (P 1 à P 20 et B 1 à B 16) et de sérums normaux (SN 10, 11, 16, 17, 18, 19).

Pour cela, les différents peptides sont adsorbés sur les microplaques à une concentration de 10 µg/ml et les sérums sont utilisés à la dilution 1/100.

Les valeurs obtenues rassemblées dans le Tableau 1 suivant correspondent à la densité optique (DO) multipliée par 10³, à 492 nm.

Pour chaque sérum, l'expérience a été réalisée en double. Les **** sont des valeurs hors échelle supérieure.

**TABLEAU 1**

| | S42G | R42Y | R40R | | S42G | P42Y | R40R |
|---|---|---|---|---|---|---|---|
| P 1 | ***** | 101 | 375 | B 1 | ***** | 869 | 172 |
| | ***** | 108 | 420 | | ***** | 811 | 173 |
| P 2 | ***** | 119 | 399 | B 2 | 2302 | 1749 | 364 |
| | ***** | 104 | 391 | | 2278 | 1664 | 345 |
| P 3 | 2145 | 648 | 223 | B 3 | 1673 | 623 | 304 |
| | 1942 | 638 | 215 | | 1686 | 630 | 341 |
| P 4 | ***** | 2314 | 309 | B 4 | ***** | 1688 | 405 |
| | ***** | 2105 | 307 | | ***** | 1557 | 346 |
| P 5 | ***** | 234 | 129 | B 5 | ***** | 1639 | 360 |
| | ***** | 243 | 176 | | 2308 | 1699 | 281 |
| P 6 | 116 | 184 | 87 | B 6 | 1671 | 810 | 172 |
| | 130 | 185 | 100 | | 1778 | 791 | 163 |
| P 7 | ***** | 2295 | 496 | B 7 | ***** | 1657 | 418 |
| | ***** | 2389 | 478 | | ***** | 1489 | 457 |
| P 8 | ***** | 983 | 282 | B 8 | 1567 | 611 | 271 |
| | ***** | 903 | 328 | | 1543 | 620 | 259 |
| P 9 | 186 | 238 | 159 | B 9 | ***** | 957 | 235 |
| | 163 | 231 | 158 | | ***** | 913 | 229 |
| P 10 | 169 | 194 | 218 | B 10 | 360 | 227 | 108 |
| | 177 | 204 | 216 | | 386 | 223 | 98 |
| P 11 | ***** | ***** | 1191 | B 11 | 1749 | 813 | 164 |
| | ***** | ***** | 1377 | | 1849 | 789 | 184 |
| P 12 | ***** | ***** | 1121 | B 12 | ***** | 755 | 136 |
| | ***** | ***** | 1231 | | ***** | 407 | 117 |
| P 13 | 114 | 64 | 113 | B 13 | 1341 | 746 | 140 |
| | 106 | 108 | 116 | | 1142 | 609 | 99 |
| P 14 | ***** | 362 | 280 | B 14 | 455 | 248 | 130 |
| | ***** | 349 | 270 | | 450 | 259 | 125 |
| P 15 | ***** | ***** | 2305 | B 15 | ***** | 313 | 301 |
| | ***** | ***** | 2335 | | ***** | 312 | 303 |
| P 16 | ***** | 1742 | 938 | B 16 | ***** | 222 | 117 |
| | ***** | 1667 | 964 | | ***** | 153 | 125 |
| P 17 | ***** | 799 | 217 | SN 10 | 205 | 237 | 163 |
| | ***** | 736 | 212 | | 192 | 205 | 154 |
| P 18 | ***** | 2253 | 1427 | SN 11 | 107 | 156 | 150 |
| | ***** | 2339 | 1327 | | 100 | 141 | 138 |
| P 19 | 105 | 106 | 84 | SN 16 | 551 | 657 | 426 |
| | 112 | 105 | 89 | | 537 | 667 | 439 |
| P 20 | ***** | 1701 | 714 | SN 17 | 129 | 156 | 104 |
| | ***** | 1679 | 740 | | 122 | 144 | 74 |
| | | | | SN 18 | 218 | 332 | 119 |
| | | | | | 173 | 279 | 87 |
| | | | | | 139 | 167 | 480 |
| | | | | SN 19 | 120 | 161 | 496 |

Le tableau 1 montre que les peptides réagissent de manière différente avec les sérums.

Il apparaît clairement que le peptide le plus réactif est le peptide S42G qui détecte 31 sérums sur 36.

Aucun de ces peptides ne détecte les sérums normaux, ce qui atteste leur spécificité.

Enfin, aucun sérum négatif avec le peptide S42G n'est positif avec les peptides P42Y ou R40R, ce qui montre qu'à lui seul le peptide S42G détecte les sérums sans l'aide des deux autres peptides.

L'étude a alors été poursuivie, afin de connaître de manière plus précise le ou les déterminants antigéniques localisés sur le peptide S42G.

Dans ce but, deux peptides ont été préparés dans les mêmes conditions que précédemment.

Ces deux peptides sont, conformément à la figure 1 :
1) un peptide de 20 acides aminés, dit S18D, couvrant la séquence 2 à 21 de la protéine CORE
2) un peptide de 24 acides aminés, dit V22G, couvrant la séquence 22 à 45 de la protéine CORE.

La réactivité de ces deux peptides (séparés et additionnés) a été évaluée en la comparant à celle du peptide S42G, dans un test ELISA tel que décrit précédemment.

La réactivité des peptides S42G, S18D, V22G, S18D + V22G, est mesurée en ELISA, vis-à-vis de sérums VHC positifs. Les différents peptides sont adsorbés sur les microplaques à une concentration de 10 µg/ml, et les sérums sont utilisés à la dilution mentionnée.

Les valeurs obtenues rassemblées dans le Tableau 2 suivant correspondent à la densité optique à 492 nm. Toutes les expériences ont été réalisées en double.

**TABLEAU 2**

| | SERUM | S42G | S18D+V22G | S18D | V22G |
|---|---|---|---|---|---|
| 1 | P1 1/100 | 2.500 | 0.438 | 0.012 | 0.426 |
| 2 | P2 1/100 | 2.500 | 0.310 | 0.020 | 0.290 |
| 3 | P3 1/100 | 2.500 | 0.665 | 0.162 | 0.503 |
| 4 | P4 1/100 | 2.500 | 2.500 | 0.982 | -1.739 |
| 5 | P5 1/100 | 2.500 | 2.500 | 2.500 | 2.500 |
| 6 | 1/1000 | 2.500 | 2.500 | 2.317 | 2.293 |
| 7 | 1/10000 | 2.093 | 0.977 | 0.399 | 0.578 |
| 8 | P6 1/100 | 0.000 | 0.007 | 0.007 | 0.000 |
| 9 | P7 1/100 | 2.500 | 2.500 | 2.500 | 2.500 |
| 10 | 1/1000 | 2.500 | 2.500 | 0.854 | 1.590 |
| 11 | 1/10000 | 1.916 | 0.402 | 0.165 | 0.237 |
| 12 | P8 1/100 | 2.500 | 2.500 | 2.500 | 2.241 |
| 13 | 1/1000 | 2.500 | 2.500 | 2.500 | 0.476 |
| 14 | 1/10000 | 1.565 | 0.730 | 0.681 | 0.049 |
| 15 | P9 1/100 | 0.090 | 0.027 | 0.019 | 0.008 |
| 16 | P10 1/100 | 0.172 | 0.054 | 0.028 | 0.026 |
| 17 | P11 1/100 | 2.500 | 2.500 | 0.383 | 2.500 |
| 18 | P12 1/100 | 2.500 | 2.500 | 2.500 | 2.500 |
| 19 | 1/1000 | 2.500 | 2.500 | 2.500 | 2.500 |
| 20 | 1/10000 | 2.500 | 0.560 | 0.454 | 0.106 |
| 21 | P13 1/100 | 0.000 | 0.025 | 0.012 | 0.013 |
| 22 | P14 1/100 | 2.500 | 2.500 | 0.907 | 1.778 |
| 23 | P15 1/100 | 2.500 | 2.500 | 2.500 | 1.810 |
| 24 | P16 1/100 | 2.500 | 2.500 | 0.225 | 2.500 |
| 25 | P17 1/100 | 2.500 | 2.129 | 0.297 | 1.832 |
| 26 | P18 1/100 | 2.500 | 2.500 | 2.500 | 2.500 |
| 27 | 1/1000 | 2.500 | 0.895 | 0.297 | 0.598 |
| 28 | 1/10000 | 1.006 | 0.167 | 0.095 | 0.072 |
| 29 | P19 1/100 | 0.000 | 0.021 | 0.011 | 0.010 |
| 30 | P20 1/100 | 2.500 | 2.500 | 1.433 | 2.249 |
| 31 | P21 1/100 | 2.500 | 2.383 | 0.111 | 2.272 |
| 32 | P22 1/100 | 2.500 | 2.500 | 2.500 | 2.500 |
| 33 | 1/1000 | 2.500 | 1.844 | 1.142 | 0.702 |
| 34 | 1/10000 | 0.894 | 0.234 | 0.146 | 0.088 |
| 35 | P23 1/100 | 0.000 | 0.030 | 0.015 | 0.015 |
| 36 | P24 1/100 | 2.500 | 0.594 | 0.015 | 0.579 |
| 37 | P25 1/100 | 2.500 | 2.500 | 2.500 | 2.252 |
| 38 | 1/1000 | 2.500 | 2.500 | 2.199 | 0.695 |
| 39 | 1/10000 | 1.550 | 0.418 | 0.329 | 0.089 |
| 40 | P26 1/100 | 2.500 | 2.500 | 2.500 | 2.500 |
| 41 | 1/1000 | 2.500 | 2.500 | 2.500 | 1.541 |
| 42 | 1/10000 | 2.500 | 1.156 | 0.957 | 0.199 |
| 43 | P27 1/100 | 2.500 | 2.500 | 1.425 | 1.600 |
| 44 | P28 1/100 | 2.500 | 2.500 | 0.115 | 2.500 |
| 45 | P29 1/100 | 0.331 | 0.005 | 0.000 | 0.005 |
| 46 | P30 1/100 | 2.500 | 2.500 | 0.483 | 2.500 |
| 47 | P31 1/100 | 2.500 | 2.500 | 2.500 | 2.500 |
| 48 | 1/1000 | 2.500 | 2.500 | 1.975 | 2.500 |
| 49 | 1/10000 | 2.071 | 1.030 | 0.183 | 0.847 |
| 50 | P32 1/100 | 2.500 | 2.500 | 1.046 | 1.639 |
| 51 | P33 1/100 | 2.500 | 2.500 | 1.307 | 1.987 |
| 52 | P34 1/100 | 2.500 | 2.500 | 2.500 | 1.618 |
| 53 | P35 1/100 | 2.500 | 2.500 | 2.500 | 1.504 |
| 54 | P36 1/100 | 2.500 | 1.341 | 0.115 | 1.226 |
| 55 | P37 1/100 | 2.500 | 2.500 | 2.500 | 2.500 |
| 56 | P1/1000 | 2.500 | 1.388 | 0.523 | 0.865 |
| 57 | 1/10000 | 1.088 | 0.230 | 0.102 | 0.128 |
| 58 | P38 1/100 | 2.500 | 2.500 | 2.500 | 2.500 |
| 59 | 1/1000 | 2.500 | 2.335 | 0.753 | 1.582 |
| 60 | 1/10000 | 1.477 | 0.156 | 0.099 | 0.057 |
| 61 | P39 1/100 | 2.500 | 2.500 | 2.500 | 1.835 |
| 62 | P40 1/100 | 2.500 | 2.500 | 2.500 | 2.500 |
| 63 | P41 1/100 | 2.500 | 2.500 | 1.579 | 2.218 |
| 64 | P42 1/100 | 0.034 | 0.000 | 0.000 | 0.000 |
| 65 | CTS 1/100 | 2.500 | 2.500 | 2.500 | 2.500 |
| 66 | 1/1000 | 2.500 | 2.500 | 2.500 | 1.155 |
| 67 | 1/10000 | 2.500 | 0.338 | 0.295 | 0.043 |
| 68 | 1/100 | 2.500 | 2.500 | 2.500 | 2.500 |
| 69 | 1/1000 | 2.500 | 2.500 | 2.500 | 1.605 |
| 70 | 1/10000 | 1.336 | 0.602 | 0.347 | 0.255 |
| 71 | B1 1/100 | 2.500 | 2.500 | 1.859 | 1.225 |
| 72 | B2 1/100 | 2.500 | 2.500 | 1.781 | 0.756 |
| 73 | B3 1/100 | 2.500 | 1.573 | 1.244 | 0.329 |
| 74 | B4 1/100 | 2.500 | 2.500 | 2.500 | 2.500 |
| 75 | 1/1000 | 2.500 | 2.464 | 1.250 | 1.214 |
| 76 | 1/1000 | 1.021 | 0.315 | 0.171 | 0.144 |
| 77 | B5 1/100 | 2.500 | 2.500 | 2.032 | 0.863 |
| 78 | B6 1/100 | 2.500 | 2.500 | 2.500 | 0.749 |
| 79 | B7 1/100 | 2.500 | 2.500 | 2.500 | 2.102 |
| 80 | B8 1/100 | 2.500 | 1.720 | 1.362 | 0.358 |
| 81 | B9 1/100 | 2.500 | 2.500 | 0.808 | 2.082 |
| 82 | B10 1/100 | 0.721 | 0.324 | 0.099 | 0.225 |
| 83 | B11 1/100 | 2.084 | 2.500 | 2.324 | 0.616 |
| 84 | B12 1/100 | 2.500 | 2.392 | 1.375 | 1.017 |
| 85 | B13 1/100 | 1.809 | 0.674 | 0.370 | 0.304 |
| 86 | B14 1/100 | 0.698 | 0.258 | 0.072 | 0.186 |
| 87 | B15 1/100 | 2.500 | 1.044 | 0.090 | 0.954 |
| 88 | B16 1/100 | 2.500 | 2.500 | 2.500 | 2.500 |

Chaque sérum a été dans un premier temps testé à la dilution 1/100. Dans le cas où la réponse s'avérait saturante (valeur 2,500) pour l'ensemble des peptides (exemple : sérum P 5), on a procédé à une dilution au 1/1000, et si besoin était à une dilution au 1/10000.

Il apparaît que pour tous les sérums VHC positifs, la réactivité du peptide S42G est nettement supérieure à la réactivité des peptides S18D et V22G, et à celle de S18D + V22G.

Les sérums P6, P9, P10, P13, P19, P23, P24 sont des sérums qui ne présentent pas d'anticorps vis-à-vis de la protéine CORE du VHC.

Bien que l'ensemble de ces résultats soit non ambigu, la fixation des différents peptides sur les puits des plaques de microtitraton, peut modifier les épitopes ou déterminants du peptide testé. Les plaques utilisées (NUNC Maxisorb) sont des plaques de polystyrène irradiées aux rayons gamma, qui fixent de façon non covalente les peptides, grâce à des liaisons de types électrostatiques mais également des liaisons hydrophobes. Il se peut que des peptides, en fonction de leur séquence, s'adsorbent de manière sélective, privilégiant ainsi une partie bien précise, et empêchant par là même la réactivité immunogénique vis-à-vis d'une autre partie qui serait devenue moins accessible.

Pour évaluer cette hypothèse, on a procédé à des tests d'inhibition, dont l'intérêt est de permettre la formation du complexe antigène-anticorps en milieu liquide, en s'affranchissant ainsi des éventuels artefacts liés à l'adsorption des peptides sur un support solide.

La méthodologie est celle décrite ci-après.

### Test d'inhibition

Les expériences d'inhibition ont été réalisées par réaction en phase liquide des sérums VHC avec les peptides, suivie de la réaction des anticorps restants avec le peptide adsorbé sur les microplaques. Les peptides inhibiteurs sont incubés à une concentration de 0,1 mg/ml, avec les sérums de la dilution appropriée. La suite de la manipulation est identique à celle décrite pour le test ELISA.

Le peptide S18D, ou V22G, ou le mélange des deux est pré-incubé une nuit, en présence du sérum à tester. Les anticorps peuvent se fixer sur les sites correspondants. Le mélange (peptide + sérum) est ensuite incubé avec le peptide S42G adsorbé sur les plaques de microtitration. Si tous les anticorps ont réagi lors de l'incubation avec les peptides S18D, V22G, ou avec le mélange, aucune réactivité ne sera observée, ce qui conduira à une inhibition de 100 %. Par opposition, si des anticorps propres au peptide S42G demeurent, ils pourront alors réagir.

Un contrôle est réalisé, en pré-incubant chaque sérum avec le peptide S42G, ce qui permet de calculer le pourcentage d'inhibition.

Le Tableau 3 rassemble les résultats de l'inhibition de la fixation des anticorps anti-VHC (dilution 1/10000) sur le peptide S42G, par préincubation de sérums VHC avec le peptide S42G, le peptide S18D, le peptide S22G et le mélange des peptides S18D + S22G.

**TABLEAU 3**

| | serum | inhibition S42G | inhibition S18D | inhibition V22G | inhibition pool S18D + V22G |
|---|---|---|---|---|---|
| 1 | P5 | 100% | 3,5% | 83,0% | 77,0% |
| 2 | P7 | 100% | 8,4% | 81,0% | 86,0% |
| 3 | P8 | 100% | 1,4% | 77,4% | 53,0% |
| 4 | P18 | 100% | 10,6% | 48,0% | 52,0% |
| 5 | P22 | 100% | 5,6% | 65,5% | 57,0% |
| 6 | P25 | 100% | 14,1% | 71,8% | 70,0% |
| 7 | P31 | 100% | 2,0% | 39,9% | 27,0% |
| 8 | P37 | 100% | 43,6% | 88,7% | 73,0% |
| 9 | P38 | 100% | 8,9% | 70,3% | 65,0% |
| 10 | B4 | 100% | 16,8% | 83,6% | 72,0% |

Comme il est montré sur le tableau 3, aucun peptide n'inhibe de façon complète la réactivité des sérums envers le peptide S42G.

Autrement dit, cette expérience prouve qu'il existe des anticorps spécifiques pour le peptide S42G, qui ne réagissent ni avec le peptide S18D, ni avec le peptide V22G, la somme des deux représentant la séquence totale du peptide S42G.

Une dernière hypothèse à évaluer consiste à vérifier que les anticorps spécifiques du peptide S42G n'étaient pas dirigés contre la partie centrale du peptide S42G, c'est-à-dire à la jonction des peptides S18D et V22G.

On a donc préparé (cf figure 1) un peptide dont la séquence comprend la partie C-terminale (6 acides aminés) du peptide S18D et la partie N-terminale (6 acides aminés) du peptide V22G.

Bien que ce peptide présente une réactivité avec des sérums VHC positifs, en aucun cas le niveau obtenu n'est comparable avec celui obtenu avec le peptide S42G.

L'ensemble des résultats présentés précédemment permet de tirer les conclusions suivantes.

Dans les 120 acides aminés N-terminaux de la protéine CORE, et plus particulièrement dans les 62 premiers acides aminés, les 45 premiers acides aminés sont les plus réactifs vis-à-vis de sérums VHC positifs.

Les 21 premiers acides aminés (peptide S18D) réagissent, ce qui montre la présence d'un ou plusieurs déterminants antigéniques sur ce peptide.

Les acides aminés 22 à 45 (peptide V22G) portent également un ou plusieurs épitopes.

La jonction de ces deux séquences est également réactive.

Par conséquent, la séquence 1-45 de la protéine CORE est pluriépitopique.

De plus, il existe un ou des déterminants antigéniques qui ne sont réactifs que dans la mesure où l'on possède la séquence 2-45 en entier, et non pas de façon discontinue (peptides S18D + V22G). Ces épitopes propres au peptide S42G sont sans aucun doute des épitopes de type conformationnel qui ne peuvent exister que dans la mesure où cette séquence de 44 acides aminés (peptide S42G) présente une structure adéquate, structure qui n'est pas obtenue avec des peptides de taille plus réduite.

Si la séquence d'acides aminés du peptide S42G ne doit pas être discontinue pour préserver tous les épitopes, on peut se demander si les parties N- et/ou C-terminales du peptide S42G interviennent dans les conformations épitopiques de S42G ou portent elles-mêmes des épitopes.

Pour tenter de répondre, on a défini cinq fragments peptidiques dérivés de S42G par des amputations N et/ou C terminales.

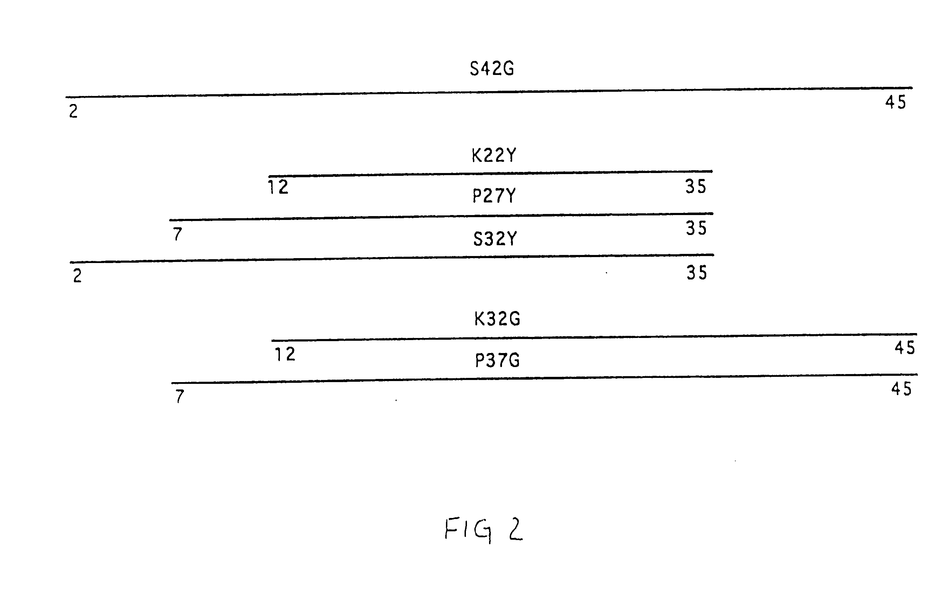

Conformément à la Figure 2, les cinq fragments suivants ont été synthétisés selon la technique de Merrifield conformément au protocole décrit précédemment :
- peptide dit P37G correspondant à la séquence d'acides aminés 7 à 45 de la protéine CORE et à une amputation de 5 acides aminés de la partie N-terminale de S42G,
- peptide dit K32G correspondant à la séquence d'acides aminés 12 à 45 de la protéine CORE et à une amputation de 10 acides aminés de la partie N-terminale de S42G,
- peptide dit S32Y correspondant à la séquence d'acides aminés 2 à 35 de la protéine CORE et à une amputation de 10 acides aminés de la partie C-terminale de S42G,
- peptide dit P27Y correspondant à la séquence d'acides aminés 7 à 35 de la protéine CORE et à une amputation de 5 acides aminés de la partie N-terminale et de 10 acides aminés de la partie C-terminale de S42G,
- peptide dit K22Y correspondant à la séquence d'acides aminés 12 à 35 de la protéine CORE et à une amputation de 10 acides aminés de la partie N-terminale et de 10 acides aminés de la partie C-terminale de S42G.

La réactivité des cinq peptides vis-à-vis de sérums d'individus infectés par le VHC a été évaluée dans des tests ELISA conformément au protocole décrit précédemment pour mesurer l'activité des peptides S42G, P42Y et R40R.

Le tableau 4 suivant rassemble les résultats obtenus pour les peptides S42G, S32Y, P27Y, K22Y pour examiner l'influence d'une amputation de la partie N-terminale et de la partie C-terminale du peptide S42G.

Ces résultats sont exprimés en valeur de densité optique lue à 492 nm multipliée par un facteur de 10³.

**TABLEAU 4**

| Sérum | dilution | RIBA C22 | S42G | S32Y | P27Y | K22Y |
|---|---|---|---|---|---|---|
| P 1 | 1/100 | | D | 703 | 620 | 435 |
| P 2 | 1/100 | | D | 1177 | 891 | 666 |
| P 3 | 1/100 | | D | 1576 | 1470 | 1031 |
| P 23 | 1/100 | | 146 | 157 | 138 | 125 |
| P 10 | 1/100 | | 56 | 78 | 60 | 66 |
| P 17 | 1/100 | | D | D | 2300 | 1930 |
| P 24 | 1/100 | | D | 1214 | 1063 | 735 |
| P 29 | 1/100 | | 594 | 400 | 347 | 310 |
| P 30 | 1/100 | | D | D | 2274 | 1805 |
| P 32 | 1/100 | | D | D | D | D |
| B 3 | 1/100 | | D | D | D | 2033 |
| P 4 | 1/100 | | D | D | D | D |
| | 1/1000 | | D | 2165 | 2111 | 1959 |
| | 1/10000 | | 588 | 369 | 324 | 300 |
| P 5 | 1/100 | | D | D | D | D |
| | 1/1000 | | D | D | D | D |
| | 1/10000 | | 1880 | 1242 | 856 | 394 |
| P7 | 1/100 | | D | D | D | D |
| | 1/1000 | | D | D | D | D |
| | 1/10000 | | 2024 | 1932 | 1667 | 1487 |
| P8 | 1/100 | | D | D | D | D |
| | 1/1000 | | D | D | D | 2257 |
| | 1/10000 | | 1423 | 1068 | 654 | 335 |
| P14 | 1/100 | | D | D | D | D |
| | 1/1000 | | D | D | D | D |
| | 1/10000 | | 1296 | 601 | 566 | 398 |
| P16 | 1/100 | | D | D | D | D |
| | 1/1000 | | D | 983 | 913 | 610 |
| | 1/10000 | | 750 | 124 | 104 | 75 |
| A8 | 1/100 | 4 | D | D | D | D |
| | 1/1000 | | D | 1372 | 1198 | 431 |
| | 1/10000 | | 620 | 173 | 118 | 56 |
| A9 | 1/100 | 4 | D | D | D | 2152 |
| | 1/1000 | | D | 1941 | 1388 | 282 |
| | 1/10000 | | 394 | 297 | 182 | 43 |
| A10 | 1/100 | 4 | D | D | 2308 | 2114 |
| | 1/1000 | | D | 373 | 334 | 207 |
| | 1/10000 | | 819 | 33 | 31 | 26 |
| A11 | 1/100 | 4 | D | 2240 | 1937 | 1878 |
| | 1/1000 | | 1865 | 228 | 226 | 212 |
| | 1/10000 | | 223 | 36 | 33 | 31 |
| A12 | 1/100 | 4 | D | D | D | D |
| | 1/1000 | | D | D | D | 646 |
| | 1/10000 | | 848 | 588 | 365 | 62 |

Les sérums P23 et P10 sont des sérums qui ne présentent pas d'anticorps vis-à-vis de la protéine CORE du VHC.

De ce tableau, on peut déduire qu'amputé des 10 acides aminés de sa partie C-terminale, S42G perd de sa réactivité et que de plus si on ampute sa partie N-terminale respectivement de 5 et 10 acides aminés, ceci se traduit par une diminution de l'immunoréactivité d'autant plus grande que la longueur du peptide est diminuée. (cf Fig 2, et en particulier les sérums P2, P5 et P8 du Tableau 4).

Le tableau 5 rassemble les résultats des tests d'immunoréactivité des peptides S42G, P37G, K32G, au cours des tests ELISA, pour examiner l'influence d'une amputation de la partie N-terminale du peptide S42G.

Les valeurs données correspondent à la densité optique lue à 492 nm multipliée par le facteur 10³.

**TABLEAU 5**

| Sérum | Dilution | S42G | P37G | K32G |
|---|---|---|---|---|
| A8 | 1/1000 | D | D | D |
| | 1/10000 | 921 | 525 | 666 |
| A9 | 1/1000 | D | 2059 | 1675 |
| | 1/10000 | 672 | 275 | 229 |
| A10 | 1/10000 | 1485 | 1215 | |
| A11 | 1/1000 | D | D | 2108 |
| | 1/10000 | 397 | 321 | 343 |
| A12 | 1/1000 | D | D | D |
| | 1/10000 | 1418 | 823 | 598 |
| A13 | 1/1000 | D | D | D |
| | 1/10000 | 1519 | 1061 | 1247 |
| A14 | 1/1000 | 1407 | 539 | 998 |
| | 1/10000 | 149 | 65 | 103 |
| A15 | 1/1000 | D | D | D |
| | 1/10000 | 1357 | 905 | 715 |
| A16 | 1/1000 | D | D | D |
| | 1/10000 | D | 2003 | D |
| A19 | 1/1000 | D | D | D |
| | 1/10000 | 620 | 446 | 594 |
| A20 | 1/1000 | D | D | D |
| | 1/10000 | D | 2338 | D |
| A21 | 1/1000 | 1319 | 652 | 993 |
| | 1/10000 | 177 | 90 | 123 |
| A22 | 1/1000 | 1216 | 702 | 876 |
| | 1/10000 | 164 | 102 | 129 |
| A23 | 1/1000 | D | D | D |
| | 1/10000 | 860 | 557 | 774 |

Ces résulats nous confortent dans l'hypothèse selon laquelle le peptide S42G doit être présent dans toute sa séquence de 2 à 45 pour présenter une immunoréactivité maximale.

Dans tous les cas S42G est supérieur à P37G, ce qui signifie que les 5 acides aminés N-terminaux jouent un rôle dans l'antigénicité.

Cependant dans certains cas, on constate peu ou pas de différence de réactivité entre les peptides P37G et K32G, ce qui tendrait à prouver que les acides aminés 7 à 11 ne sont pas d'une importance majeure pour l'antigénicité du peptide S42G.

Par ailleurs, la comparaison des Tableaux 4 et 5 permet de mettre en évidence l'importance des 10 acides aminés C-terminaux du peptide S42G dans l'immunoréactivité de celui-ci.

Pour terminer, on a substitué la protéine CORE entière (191 acides aminés) par le peptide S42G (44 acides aminés), pour détecter les anticorps anti VHC.

Pour ce faire, on a choisi de comparer la sensibilité du peptide S42G à celle du test ORTHO VHC ELISA 2è génération ; il s'agit d'un test commercialisé par la Société ORTHO comprenant une protéine de fusion incorporant la protéine CORE du VHC, nommée C22-3 ; cf Vanderpoel, C.L, HTM Cuypers, H.W Reesink et al, 1991, Confirmation of hepatites C virus infection by new four antigen recombinant immunoblot assay, Lancet 337 ; 317-319.

La comparaison a porté sur 173 échantillons positifs avec le test ORTHO VHC ELISA 2è génération.

Sur 173 échantillons, le peptide S42G en a détecté 151, ce qui donne une sensibilité de 87,28 %. Les 22 sérums discordants ont alors été analysés à l'aide d'un autre test de 2è génération, à savoir CHIRON RIBA VHC. Il s'agit d'un "immunoblot" destiné à la détection des anticorps dirigés contre les antigènes du virus de l'hépatite C dans le sérum ou le plasma humain. Ce test comprend cinq antigènes (protéines) recombinants. L'un d'entre eux est la protéine CORE recombinante C22-3, obtenue sous forme de protéine de fusion avec la superoxyde dismutase humaine, et exprimée par une levure.

Il s'avère à l'issue de ce test de confirmation, qu'aucun des 22 sérums ne présente de réactivité vis-à-vis de la bande C22-3 (CORE).

En conséquence, la sensibilité du peptide S42G est de 100 % par rapport à la protéine CORE (C22-3) du test CHIRON RIBA VHC de 2è génération.

En conclusion, on peut remplacer la protéine CORE par le peptide S42G, pour la détection sérologique du VHC.

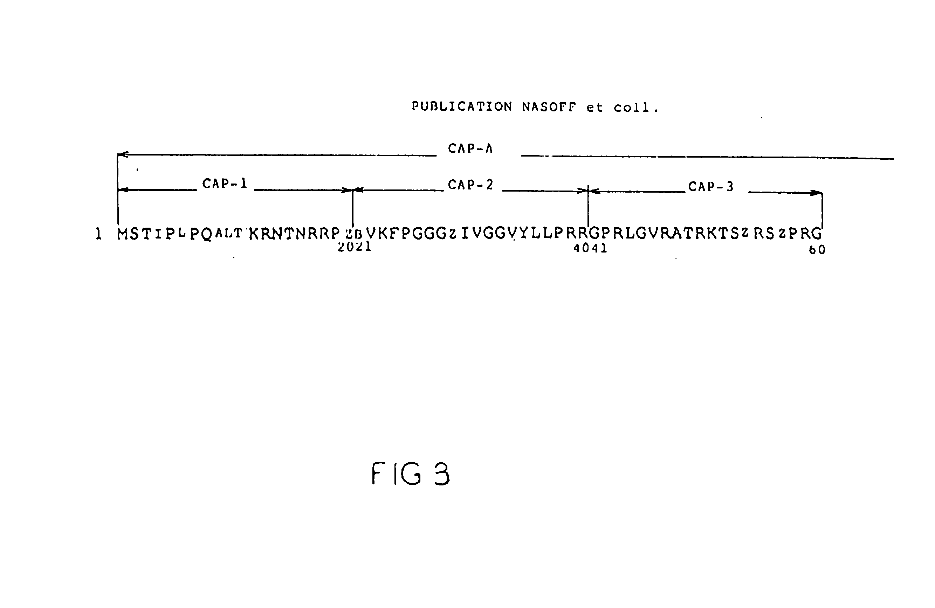

A ce stade de l'exposé de l'invention, il convient de mettre en évidence l'utilisation avantageuse de peptides de synthèse par rapport à celle de fragments protéiques recombinants. En cela, on a comparé les résultats et constatations expérimentales selon l'invention, avec ceux de la publication, à savoir : Nasoff MS, Zebedee SL, Inchauspe G, Prince AM, Identification of an immunodominant epitope within the capsid protein of hepatis C virus, Proc Natl Acad Sci USA 1991 ; 88 : 4641-5. Cette publication est relative à l'obtention de fragments protéiques recombinants de la protéine CORE exprimée dans E. coli, conformément à la Figure 3, et rapporte des résultats à la fois proches et divergents de ceux rapportés précédemment.

En effet, les auteurs ont exprimé une protéine recombinante comprenant les 74 premiers acides aminés de la protéine CORE. La stratégie de clonage utilisée conduit à la production de protéines de fusion. Autrement dit, les 74 acides aminés N-terminaux de la protéine CORE du VHC dits CAP-A, sont précédés par 308 acides aminés, dont les 221 premiers correspondent à la glutathione S-transférase. La réactivité de cette protéine de 382 acides aminés, dont seulement 20 % représentent la protéine CORE, vis-à-vis de sérums VHC positifs n'est bonne qu'en apparence, compte tenu du nombre très faible de sérums testés (5 sérums humains).

Par contre, une protéine comprenant la séquence 69-120 de la protéine CORE, dite CAP-B, ne présente aucune réactivité vis-à-vis de ces mêmes sérums. Ce dernier résultat est en contradiction relative avec ceux de la présente invention, puisque le peptide R40R qui comprend la séquence 75-116 de cette même protéine réagit tout de même avec quelques sérums (environ 10 %, cf tableau 1).

Ces mêmes auteurs ont poursuivi leur travail, en produisant d'autres protéines recombinantes de fusion, avec respectivement comme séquence de la protéine CORE, les acides aminés 1-20, 21-40, 41-60, dits respectivement CAP-1, CAP-2 et CAP-3.

Leurs résultats toujours obtenus sur un nombre de sérums humains très faibles (9 sérums), montrent que la séquence 21-40 réagit mieux que la séquence 1-20. La séquence 41-60 ne présente quant à elle aucune réactivité. Ce dernier résultat est également en contradiction relative avec ceux présentés selon l'invention, puisque le peptide P42Y (acides aminés 39-82) présente (cf tableau 1), une réactivité élevée bien qu'inférieure au peptide S42G.

De plus, une autre publicaton à savoir :
Okamoto H, Munekata E, Tsuda F, Takahashi K, Yotsumoto C, et al, 1990, Jpn, J. Exp Med 60, 223-233, a montré qu'un peptide de 36 acides aminés comprenant la séquence 39-74 de la protéine CORE réagit avec au moins 70 % des sérums VHC positifs.

Concernant la réactivité de la séquence 21-40, ces auteurs affirment que dans plusieurs cas la réactivité de ce fragment de protéine CORE recombinant est supérieure à celui comprenant la séquence 1-74.

Les résultats obtenus selon l'invention sont en désaccord avec les résultats de ces auteurs, puisqu'on a démontré que dans tous les cas de sérums VHC étudiés (cf tableau 2), la réactivité du peptide S42G (séquence 2-45) est nettement supérieure à celle des peptides S18D (séquence 2-21) et V22G (séquence 22-45).

L'explication proposée pour expliquer ces résultats divergents concerne l'obtention des différents fragments de la protéine CORE dans les deux cas.

Selon l'invention, les peptides obtenus par synthèse chimique comprennent uniquement la séquence mentionnée pour chacun d'entre eux, et comme il a été explicité plus haut, ceci est un des avantages liés à ces peptides synthétiques.

En ce qui concerne les protéines recombinantes obtenues par NASOFF et al., il s'agit de protéines de fusion dans lesquelles se trouvent en position N-terminale 308 acides aminés qui sont totalement étrangers de la protéine CORE.

Les protéines recombinantes qui comprennent les séquences 1-20 et 21-40 de la protéine CORE sont donc à plus de 90 % composées d'une séquence étrangère.

Bien que les auteurs stipulent que pour des tests de détection, la partie glutathione -S-transférase de la protéine de fusion ne gène pas la réaction, puisqu'aucun des sérums testés ne réagit avec la glutathione S-transférase isolée (donc pas de faux positif), il apparaît difficile d'admettre que ces 90 % de protéine de fusion n'interfèrent en aucune manière dans la réactivité avec les anticorps anti-VHC.

En effet, le fait que la partie N-terminale des séquences 1-20 ou 21-40 de la protéine CORE soit liée à la partie C-terminale de la protéine de fusion contribue à restreindre l'accessibilité de cette région N-terminale. Par contre, la partie C-terminale est quant à elle mise en évidence. C'est très vraisemblablement ces contraintes structurales, imposées par l'obtention d'une protéine recombinante de fusion dans laquelle la partie immunogène (la partie CORE) représente la partie mineure de la protéine de fusion (moins de 10 % dans ce cas), qui conduisent à des résultats inverses à ceux présentés.

Les acides aminés sont représentés selon les Figures 1 et 2, selon la convention du tableau ci-après :

**TABLEAU 6**

| ACIDE AMINE | CODE 3 LETTRES | POIDS MOLECULAIRE |
|---|---|---|
| ALANINE | Ala | 89 |
| CYSTEINE | Cys | 121 |
| AC. ASPARTIQUE | Asp | 133 |
| AC. GLUTAMIQUE | GlU | 147 |
| PHENYLALANINE | Phe | 165 |
| GLYCINE | Gly | 75 |
| HISTIDINE | His | 155 |
| ISOLEUCINE | Ile | 131 |
| LYSINE | Lys | 146 |
| LEUCINE | Leu | 131 |
| METHIONINE | Met | 149 |
| ASPARAGINE | Asn | 132 |
| PROLINE | Pro | 115 |
| GLUTAMINE | Gln | 146 |
| ARGININE | Arg | 174 |
| SERINE | Ser | 105 |
| THREONINE | Thr | 119 |
| VALINE | Val | 117 |
| TRYPTOPHANE | Trp | 204 |
| TYROSINE | Tyr | 181 |

Selon l'invention le protocole expérimental complet, exposé précédemment, démontre clairement par des types de réaction antigène-anticorps réalisés soit en phase solide (ELISA direct, cf Tableau 2), soit en inhibition (cf Tableau 3), que la séquence 2-45 de la protéine CORE du virus VHC obtenu par synthèse chimique en phase solide, non seulement s'avère nettement supérieure à des séquences plus petites (S18D ou V22G), mais également qu'elle présente une sensibilité équivalente à la protéine CORE elle-même (protéine C22-3 du test CHIRON RIBA VHC de 2ème génération), et qu'en conséquence on peut utiliser dans des tests diagnostiques sérologiques le peptide synthétique S42G, en lieu et place de la protéine CORE.

Il ressort de tous ces résultats que le peptide S42G apparaît être la structure minimale mais suffisante qui du point de vue de ses propriétés antigéniques est équivalente à la protéine CORE dans sa totalité et peut donc la remplacer dans un réactif de détection du VHC.

### NOMBRE DE SEQUENCES 2

1) INFORMATION CONCERNANT SEQ ID N01:
   i) CARACTERISTIQUES DE LA SEQUENCE
      A) LONGUEUR : 45 acides aminés
      B) TYPE : séquence d'acides aminés
   ii) TYPE DE MOLECULE : peptide
   iii) HYPOTHETIQUE : non
   v) TYPE DE FRAGMENT : N-terminal
   vi) ORIGINE :
      A) ORGANISME : virus humain de l'hépatite C
      B) SOUCHE : H77 publiée selon Ogata N. et al, Nucleotide Sequence and Mutation Rate of the H strain of Hepatitis Virus, Proc. Natl. Academ Sci. USA, 88:3392-6 (1991)
   ix) CARACTERISTIQUES :
      A) NOM/CLE : peptide
      B) EMPLACEMENT : 1..45
      D) AUTRES INFORMATIONS :
         séquence N-terminale de la protéine de nucléocapside ou protéine CORE du virus humain de l'hépatite C.
   xi) DESCRIPTION DE LA SEQUENCE : SEQ ID N01 
Met Ser Thr Asn Pro Lys Pro Gln Arg Lys Thr Lys Arg Asn Thr Asn
 1               5                   10                  15
Arg Arg Pro Gln Asp Val Lys Phe Pro Gly Gly Gly Gln Ile Val Gly
            20                  25                  30
Gly Val Tyr Leu Leu Pro Arg Arg Gly Pro Arg Leu Gly
        35                  40                  45
2) INFORMATION CONCERNANT SEQ ID N02:
   i) CARACTERISTIQUES DE LA SEQUENCE:
      A) LONGUEUR : 44 acides aminés
      B) TYPE : séquence d'acides aminés
   ii) TYPE DE MOLECULE : peptide
   iii) HYPOTHETIQUE : non
   v) TYPE DE FRAGMENT : N-terminal
   vi) ORIGINE :
      A) ORGANISME : virus humain de l'hépatite C
      B) SOUCHE : H77 publiée selon Ogata N. et al, Nucleotide Sequence and Mutation Rate of the H strain of Hepatitis Virus, Proc. Natl. Academ Sci. USA, 88:3392-6 (1991)
   ix) CARACTERISTIQUES :
      A) NOM/CLE : peptide
      B) EMPLACEMENT : 2..45
      D) AUTRES INFORMATIONS :
         séquence N-terminale de la protéine de nucléocapside ou protéine CORE du virus humain de l'hépatite C.
   xi) DESCRIPTION DE LA SEQUENCE : SEQ ID N02

Ser Thr Asn Pro Lys Pro Gln Arg Lys Thr Lys Arg Asn Thr Asn Arg
 2           5                   10                  15
Arg Pro Gln Asp Val Lys Phe Pro Gly Gly Gly Gln Ile Val Gly Gly
        20                  25                  30
Val Tyr Leu Leu Pro Arg Arg Gly Pro Arg Leu Gly
    35                  40                  45

## Revendications

1. Polypeptide consistant en une séquence peptidique présentant une réactivité immunologique vis-à-vis du VHC et choisie parmi les séquences identifiées par SEQ ID NO :1 et SEQ ID NO :2, et les séquences homologues à SEQ ID NO :1 et SEQ ID NO :2.

2. Composition polypeptidique, **caractérisée en ce qu'**elle comprend en mélange au moins deux polypeptides différents, dont la séquence présente une activité immunologique vis-à-vis du VHC et est choisie parmi (a) les séquences SEQ ID NO :1 et SEQ ID NO :2, (b) les séquences correspondant à une amputation de SEQ ID NO :1, dans sa partie N-terminale de 1 à 10 acides aminés ou dans sa partie C-terminale de 1 à 10 acides aminés, et (c) les séquences homologues aux séquences selon (a) ou (b).

3. Composition polypeptidique selon la revendication 2, **caractérisée en ce que** la séquence selon (b) se compose de SEQ ID NO : 1 amputée de 1 à 10 acides aminés dans sa partie C-terminale.

4. Composition polypeptidique selon la revendication 2, **caractérisée en ce que** la séquence selon (b) se compose de SEQ ID NO : 1 amputée de 1 à 10 acides aminés dans sa partie N-terminale.

5. Réactif de détection du virus humain de l'hépatite C (VHC), **caractérisé en ce qu'**il comprend à titre de substance réactive, un polypeptide selon la revendication 1, ou une composition selon l'une quelconque des revendications 2 à 4.

6. Moyen de détection du virus humain de l'hépatite C (VHC), **caractérisé en ce qu'**un réactif selon la revendication 5, est supporté par un support solide, immunologiquement compatible avec ledit réactif.

7. Procédé de détection d'anticorps anti-VHC dans une partie corporelle, telle qu'un échantillon du sang d'un individu suspecté d'être infecté par le VHC, **caractérisé en ce qu'**on met en contact ladite partie corporelle et un réactif selon la revendication 5, dans des conditions permettant une éventuelle réaction immunologique, et on détecte ensuite la présence d'un complexe immun avec ledit réactif.

8. Dispositif ou appareil de détection d'anticorps anti-VHC dans une partie corporelle, telle qu'un échantillon du sang d'un individu suspecté d'être infecté par le VHC, **caractérisé en ce qu'**il comprend un récipient de mise en contact de ladite partie corporelle avec un réactif selon la revendication 5, avec des moyens. créant des conditions, telles que température, adaptés à une éventuelle réaction immunologique, et des moyens de détection, notamment optiques, d'un complexe immun avec ledit réactif.

9. Réactif de détection d'un polypeptide selon la revendication 1, **caractérisé en ce qu'**il comprend à titre de substance active des anticorps monoclonaux ou polyclonaux dirigés contre le virus humain de l'hépatite C, obtenus par réaction immunologique d'un organisme humain ou animal à un agent immunogène constitué par un polypeptide selon la revendication 1.

## Claims

1. Polypeptide consisting of a peptide sequence exhibiting immunological reactivity towards HCV and chosen from the sequences identified by SEQ ID NO1 and SEQ ID NO2, and the sequences homologous to SEQ ID NO1 and SEQ ID NO2.

2. Polypeptide composition, **characterized in that** it comprises, in a mixture, at least two different polypeptides whose sequence exhibits immunological reactivity towards HCV and is chosen from (a) the sequences SEQ ID NO1 and SEQ ID NO2, (b) the sequences corresponding to an amputation of SEQ ID NO1, of 1 to 10 amino acids from its N-terminal part or of 1 to 10 amino acids from its C-terminal part, and (c) the sequences homologous to the sequences according to (a) or (b).

3. Polypeptide composition according to Claim 2, **characterized in that** the sequence according to (b) is composed of SEQ ID NO1 from which 1 to 10 amino acids are amputated at its C-terminal end.

4. Polypeptide composition according to claim 2, **characterized in that** the sequence according to (b) is composed of SEQ ID NO1 from which 1 to 10 amino acids are amputated from its N-terminal end.

5. Reagent for the detection of human hepatitis C virus (HCV), **characterized in that** it comprises as reactive substance a polypeptide according to Claim 1, or a composition according to any one of Claims 2 to 4.

6. Means for the detection of human hepatitis C virus (HCV), **characterized in that** a reagent according to Claim 5 is supported by a solid support immunologically compatible with the said reagent.

7. Process for the detection of anti-HCV antibodies in a body part, such as a blood sample of an individual suspected of being infected with HCV, **characterized in that** the said body part and a reagent according to Claim 5 are brought into contact under conditions which permit an immunological reaction where appropriate and the presence of an immune complex is then detected with the said reagent.

8. Device or apparatus for the detection of anti-HCV antibodies in a body part, such as a blood sample of an individual suspected of being infected with HCV, **characterized in that** it comprises a vessel for bringing the said body part into contact with a reagent according to Claim 5, with means which create conditions, such as temperature, suitable for an immunological reaction where appropriate, and means, especially optical, for the detection of an immune complex with the said reagent.

9. Reagent for the detection of a polypeptide according to claim 1 **characterized in that** it comprises as reactive substance, monoclonal or polyclonal antibodies directed against the human hepatitis C virus, that are obtained by immunological reaction of a human or animal organism to an immunogenic agent consisting of a polypeptide according to Claim 1.

## Patentansprüche

1. Polypeptid, bestehend aus einer Peptidsequenz, die eine immunologische Reaktivität gegenüber dem HCV aufweist und aus den Sequenzen ausgewählt ist, die durch SEQ ID-NO. 1 und SEQ ID-NO. 2 sowie die homologen Sequenzen von SEQ ID-NO. 1 und SEQ ID-NO. 2 identifiziert sind.

2. Polypeptidmischung, **dadurch gekennzeichnet, daß** sie eine Mischung aus mindestens zwei verschiedenen Polypeptiden aufweist, deren Sequenz eine immunologische Reaktivät genenüber dem HCV aufweist und ausgewählt ist aus (a) den Sequenzen SEQ ID-NO. 1 und SEQ ID-NO. 2, (b) den Sequenzen, die einer Kürzung von SEQ ID-NO. 1 um 1 bis 10 Aminosäuren in ihrem N-terminalen Bereich oder um 1 bis 10 Aminosäuren in ihrem C-terminalen Bereich entsprechen, and (c) den Sequenzen. die homolog zu den Sequenzen gemäß (a) oder (b) sind.

3. Polypeptidmischung nach Anspruch 2, **dadurch gekennzeichnet, daß** sich die Sequenz gemäß (b) aus der um 1 bis 10 Aminosäuren an ihrem C-terminalen Ende gekürzten SEQ ID-NO. 1 zusammensetzt.

4. Polypeptidmischung nach Anspruch 2, **dadurch gekennzeichnet, daß** sich die Sequenz gemäß (b) aus der um 1 bis 10 Aminosäuren an ihrem N-terminalen Ende gekürzten SEQ ID-NO. 1 zusammensetzt.

5. Reagenz zum Nachweis von humanem Hepatitis-C-Virus (HCV), **dadurch gekennzeichnet, daß** es als reaktive Substanz ein Polypeptid nach Anspruch 1 oder eine Mischung nach einem der Anspruche 2 bis 4 aufweist.

6. Mittel zum Nachweis des humanen Hepatitis-C-Virus (HCV), **dadurch gekennzeichnet, daß** ein Reagenz nach Anspruch 5 von einem festen Träger getragen wird, der immunologisch mit dem besagten Reagenz vereinbar ist.

7. Verfahren zum Nachweis von Anti-HCV-Antikörpern in einem Teil des Körpers, wie z.B. einer Blutprobe eines Individuums, das in Verdacht steht, mit HCV infiziert zu sein, **dadurch gekennzeichnet, daß** man den besagten Teil des Körpers and ein Reagenz nach Anspruch 5 unter Bedingungen in Kontakt bringt, die eine eventuelle immunologische Reaktion erlauben, und daß man anschließend die Anwesenheit eines Immunkomplexes mit dem besagten Reagenz nachweist.

8. Vorrichtung oder Gerät zum Nachweis von Anti-HCV-Antikörpern in einem Teil des Körpers, wie z.B. einer Blutprobe eines Individuums, das in Verdacht steht, mit HCV infiziert zu sein **dadurch gekennzeichnet, daß** sie/es ein Behältnis aufweist, urn den besagten Teil des Körpers mit einem Reagenz nach Anspruch 5 in Kontakt zu bringen, mit Mitteln, die Bedingungen, wie beispielsweise Temperatur schaffen, die an eine eventuelle immunologische Reaktion angepaßt sind, and Mitteln, insbesondere optischen Mitteln zum Nachweis eines Immunkomplexes mit besagtem Reagenz.

9. Reagenz zum Nachweis eines Polypeptids nach Anspruch 1 **dadurch gekennzeichnet, daß** es als wirksame Substanz monoklonale oder polyklonale gegen das humane Hepatitis-C-Virus gerichtete Antikörper, daß sie durch eine immunologische Reaktion eines humanen oder tierischen Organismus auf ein immunogenes Reagenz gewonnen wurden, das aus einem Polypeptid nach Anspruch 1 aufgebaut ist.
